# EUROPEAN PATENT SPECIFICATION

(11) **EP 4 380 395 B1**
(45) Date of publication and mention of the grant of the patent: **06.05.2026**
(21) Application number: 22758231.9
(22) Date of filing: 01.08.2022
(51) Int. Cl.: A24F 40/51, A61B 5/08

(54) **AEROSOL GENERATION DEVICE COMPRISING A GAS SENSOR AND ASSOCIATED AEROSOL GENERATION ASSEMBLY**
AEROSOLERZEUGUNGSVORRICHTUNG MIT EINEM GASSENSOR UND ZUGEHÖRIGE AEROSOLERZEUGUNGSANORDNUNG
DISPOSITIF DE GÉNÉRATION D'AÉROSOL COMPRENANT UN DÉTECTEUR DE GAZ, ET ENSEMBLE DE GÉNÉRATION D'AÉROSOL ASSOCIÉ

(30) Priority: 02.08.2021 EP 21189154
(43) Date of publication of application: 12.06.2024
(73) Proprietor: JT International S.A., 1202 Genève (CH)
(72) Inventor: YAMAGUCHI, Akira, 1207 GENEVE (CH)
(74) Representative: Lavoix
(86) International application number: PCT/EP2022/071553
(87) International publication number: WO 2023/012104

(56) References cited:
- WO-A1-2019/175810
- WO-A1-2020/141128
- WO-A1-2020/209798
- US-A1- 2016 255 878

## Description

### FIELD OF THE INVENTION

The present invention concerns an aerosol generation device.

The present invention also concerns an aerosol generation assembly comprising such an aerosol generation device and a consumable article.

### BACKGROUND OF THE INVENTION

Different types of aerosol generation devices are already known in the art. Generally, such aerosol generation devices comprise a storage portion for storing a vaporizable material or for receiving a consumable article storing such a vaporizable material. The vaporizable material can comprise for example a liquid or a solid. A heating system is formed of one or more electrically activated resistive heating elements arranged to heat said vaporizable material to generate the aerosol. The aerosol is released into a flow path extending between an inlet and an outlet of the aerosol generation device.

US 2016/0255878 A1 discloses a smart electronics vaporizer device.

Generally, aerosol generation devices are designed so as to be easy to carry for a user. Typically, a user carries the aerosol generation device most of the time throughout the day to be able to use it at any time.

Typically, such aerosol generation devices are used regularly throughout the day. For instance, a smoker commonly uses such a device during breaks or after meals, for example, to relax, relieve stress and/or anxiety. These devices are used when or after changes occur in the user's health and wellness. However, the user can hardly realize the extent of said changes.

Monitoring said changes could greatly help to improve a user's knowledge on her/his own physical and psychological condition. Therefore, there is a need for an easy way to provide a user with data about her/his health and wellness.

### SUMMARY OF THE INVENTION

One of the aims of the invention is to provide an aerosol generation device that provides better knowledge about its user's wellness.

For this purpose, the invention relates to an aerosol generation device comprising:
- a storage portion configured to receive a consumable article storing a vaporizable material, the storage portion extending along a device axis between an open end and a bottom end;
- a first heating system configured to heat at least partially the storage portion until at least a first temperature to form aerosol on the open end when the consumable article is received in the storage portion;
- a gas-sensing compartment, able to be in fluid communication with the storage portion and comprising a gas sensor for sensing at least a gas;

in a vaping mode, the storage portion being configured to conduct a vaping flow from a vaping flow inlet to the open end through the consumable article;
in a sensing mode, the storage portion being configured to conduct an exhaled flow from the open end to the gas-sensing compartment.

Aerosol generation devices are used during particular moments of the day during which specific changes occur in the user's metabolism. For instance, a user may use an aerosol generation device after eating food which drastically modifies her/his metabolism. During breaks, a user may use the device to relax. Feelings and mood also impact the metabolism of the user. The gas sensor of the device is used to sense at least a gas exhaled by the user. For instance, the aerosol generation device offers a way to analyze the user's metabolism and provide information about it. Monitoring a user's metabolic parameters greatly helps to improve her/his health and wellness by providing her/him with crucial information and enabling her/him to take actions accordingly.

According to some embodiments, the aerosol generation device further comprises a sensing valve arranged between the gas-sensing compartment and the storage portion;
the sensing valve being movable between an open position in which the gas-sensing compartment is in fluid communication with the storage portion and a closed position in which the gas-sensing compartment is isolated from the storage portion;
the sensing valve being in the open position in the sensing mode and in the closed position in the vaping mode.

According to some embodiments, the aerosol generation device further comprises a vaping valve arranged between the vaping flow inlet and the storage portion;
the vaping valve being movable between an open position in which the vaping flow inlet is in fluid communication with the storage portion and a closed position in which the vaping flow inlet is sealed;
the vaping valve being in the open position in the vaping mode and in the closed position in the sensing mode.

Thanks to these features, the device can be used between a classic vaping mode and a sensing mode in which the device analyze the gas exhaled by the user. This simple fluidic circuit allows the device to switch between both modes in a simple manner.

According to some embodiments, the aerosol generation device further comprises a control module and a switch system, the control module being configured to control the device between the sensing mode and the vaping mode, the switch system being designed to be manipulated by a user to command the control module.

Thanks to these features, the user can easily select a desired mode.

According to some embodiments, the storage portion is configured to receive the consumable article through the open end.

According to some embodiments, in the sensing mode, the storage portion is configured to conduct the exhaled flow when the storage portion is empty of consumable article.

According to some embodiments, the vaping flow inlet is arranged at the bottom end of the storage portion.

Thanks to these features, common parts of the aerosol generation device are used to convey the exhaled flow to the gas-sensing compartment. The manufacturing of the device remains easy.

According to some embodiments, the first heating system is further configured to heat the exhaled flow passing through the storage portion in the sensing mode, until at least a second temperature.

According to some embodiments, the aerosol generation device further comprises a second heating system configured to heat the exhaled flow entering the gas-sensing compartment in the sensing mode, until at least a second temperature.

Thanks to these features, the exhaled flow is heated in order to make the analysis carried out by the gas sensor easier.

According to some embodiments, the first heating system and/or the second heating system comprises a hollow cylindrical component, the exhaled flow flowing through the cylindrical component toward the gas sensor in the sensing mode.

According to some embodiments, the cylindrical component comprises, radially, inwardly to outwardly, a Teflon layer filled with a hydrocarbon filler, a copper layer and a heater film.

Thanks to these features, the heating systems remain simple and efficient. The hollow cylindrical component accounts for a part of the circuit conveying the exhaled flow to the gas sensor. The copper layer enables a good thermal conduction of the heat generated by the heater film.

According to some embodiments, the second temperature is chosen to cause isolation of a at least one predetermined component from the exhaled flow, preferably the second temperature being comprised between 80°C and 100°C, and advantageously substantially equal to 90°C.

Thanks to these features, the analysis carried out by the sensor is as accurate as possible with respect, for example, to a specific gas component.

According to some embodiments, the aerosol generation device further comprises a communication module connected to the gas sensor, the gas sensor being configured to generate sensing data representative of the exhaled flow, the communication module being configured to transmit said sensing data to an external device.

Thanks to these features, sensing data generated by the aerosol generation device are conveyed to an external device. For example, said data are then displayed in a simple manner so that the user can acknowledge information about its metabolism.

According to some embodiments, the gas sensed by the gas sensor comprises a component chosen from the list comprising acetone, carbon dioxide and ethanol.

Thanks to these features, the user can have access to information about key components of the human metabolism.

The invention also relates to an aerosol generation assembly comprising:
- an aerosol generation device as described above; and
- a consumable article configured to operate with the aerosol generation device.

### BRIEF DESCRIPTION OF THE DRAWINGS

- Figure 1 is an illustration of an aerosol generation device according to the invention and of an external device, the aerosol generation device being in sensing mode and transmitting sensing data to the external device;
- Figure 2 is a cross-section representation of an aerosol generation assembly according to the invention, the aerosol generation assembly comprising an aerosol generation device of Figure 1 and a consumable article inserted into a storage portion of this device, the device being in vaping mode; and
- Figure 3 is a cross-section representation of the aerosol generation device of Figure 2 without the consumable article, the device being in sensing mode.

### DETAILED DESCRIPTION OF THE INVENTION

Before describing the invention, it is to be understood that it is not limited to the details of construction set forth in the following description. It will be apparent to those skilled in the art having the benefit of the present disclosure that the invention is capable of other embodiments and of being practiced or being carried out in various ways.

As used herein, the term "aerosol generation device" or "device" may include a vaping device to deliver an aerosol to a user, including an aerosol for vaping, by means of aerosol generating unit (e.g. an aerosol generating element which generates vapor which condenses into an aerosol before delivery to an outlet of the device at, for example, a mouthpiece, for inhalation by a user). The device may be portable. "Portable" may refer to the device being for use when held by a user. The device may be adapted to generate a variable amount of aerosol, e.g. by activating a heater system for a variable amount of time (as opposed to a metered dose of aerosol), which can be controlled by a trigger. The trigger may be user activated, such as a vaping button and/or inhalation sensor. The inhalation sensor may be sensitive to the strength of inhalation as well as the duration of inhalation to enable a variable amount of vapor to be provided (so as to mimic the effect of smoking a conventional combustible smoking article such as a cigarette, cigar or pipe, etc.). The device may include a temperature regulation control to drive the temperature of the heater and/or the heated aerosol generating substance (aerosol pre-cursor) to a specified target temperature and thereafter to maintain the temperature at the target temperature that enables efficient generation of aerosol.

As used herein, the term **"aerosol"** may include a suspension of vaporizable material as one or more of: solid particles; liquid droplets; gas. Said suspension may be in a gas including air. Aerosol herein may generally refer to/include a vapor. Aerosol may include one or more components of the vaporizable material.

As used herein, the term **"vaporizable material"** or **"precursor"** or **"aerosol forming substance"** or **"substance"** is used to designate any material that is vaporizable in air to form aerosol. Vaporization is generally obtained by a temperature increase up to the boiling point of the vaporization material, such as at a temperature less than 400°C, preferably up to 350°C. The vaporizable material may, for example, comprise or consist of an aerosol-generating liquid, gel, wax, foam or the like, an aerosol-generating solid that may be in the form of a rod, which contains processed tobacco material, a crimped sheet or oriented strips of reconstituted tobacco (RTB), or any combination of these. The vaporizable material may comprise one or more of: nicotine, caffeine or other active components. The active component may be carried with a carrier, which may be a liquid. The carrier may include propylene glycol or glycerin. A flavoring may also be present. The flavoring may include Ethylvanillin (vanilla), menthol, Isoamyl acetate (banana oil) or similar.

As used herein, the term **"external device"** may refer to a device, which is able to establish a wireless data connection with the aerosol generation device as it is explained in the specification. Such an external device may be a mobile device like a mobile phone for example. Additionally, such an external device may be a smart device able to process at least some data received from the aerosol generation device or intended to be transmitted to the aerosol generation device. Such a smart device can be a smartphone, a smartwatch, a tablet computer, a laptop, a desktop computer or any other smart object implemented for example according to the loT ("Internet of things") technology.

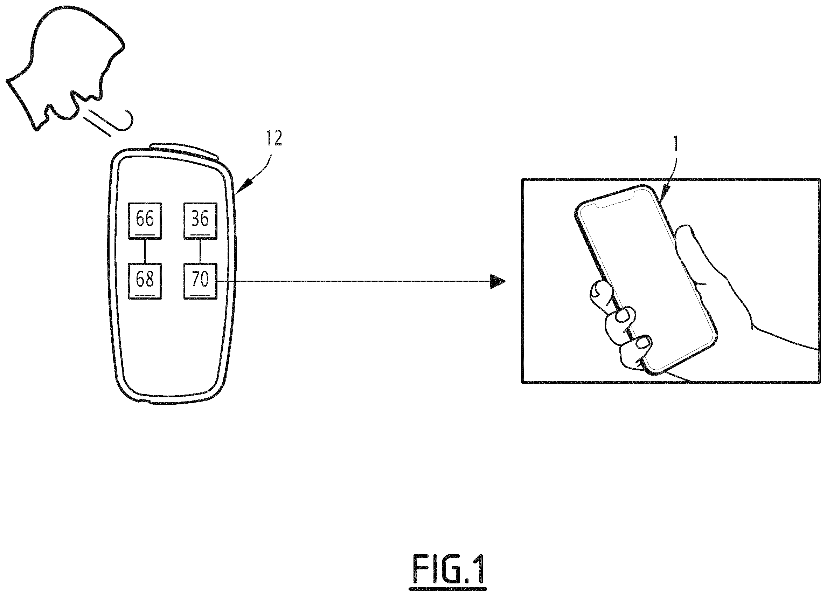

An aerosol generation assembly 10 is shown on Figure 1. For example, the aerosol generation assembly 10 is designed to operate with an external device 1, as illustrated on Figure 1.

The aerosol generation assembly 10 comprises an aerosol generation device 12 and a consumable article 14 configured to operate with the aerosol generation device 12.

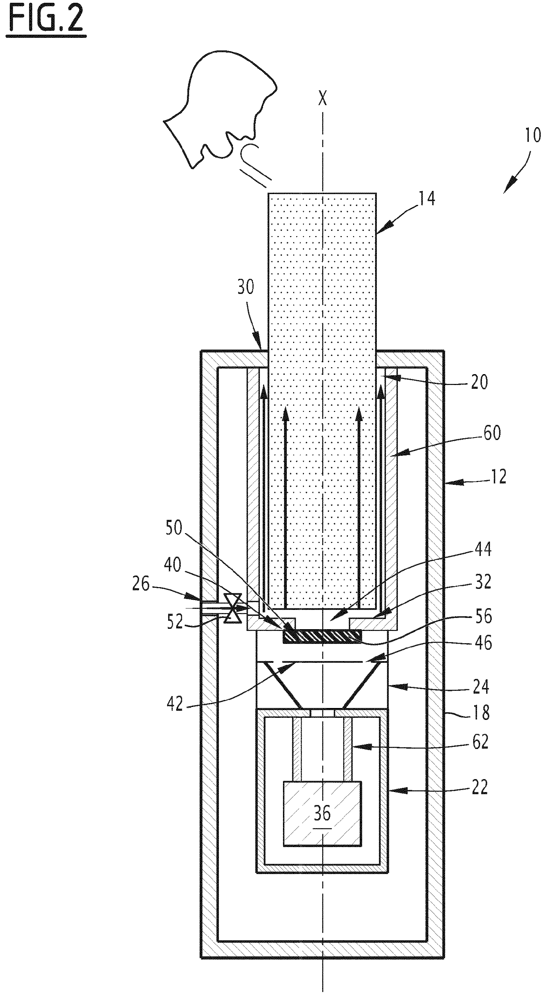

The consumable article 14 stores a vaporizable material. In the particular example shown on Figure 2, the consumable article 14 is a stick or a tobacco rod having a cylindrical shape and a circular or elliptical cross-section. The stick may have a length comprised between 69 mm and 100 mm and a diameter comprised between 5 mm and 8 mm. As a variant, the consumable article 14 may have a different shape. For example, the stick is a ready-made cigarette. According to another example, the stick presents a flat-shape and a rectangular cross-section. According to still another example, the consumable article 14 may contain a vaporizable material in liquid form.

The consumable article 14 may have a mouthpiece portion designed to be in contact with the user's mouth/lips and a vaporizable material containing portion designed to store the vaporizable material. This vaporizable material containing portion is designed to be heated by a heater as it will be explained below in further detail or to be at least in fluid communication with the heater. The heater is configured to heat the vaporizable material until at least a heating temperature and may be at least partially integrated in the consumable article 14 and/or device 12. In the example of the Figures, the heater in entirely integrated in the device 12. The heating temperature of the vaporizable material depends on the nature of the heating material and may for example be less than 400°C and is preferably comprised between 200°C and 390°C. More generally, in case of a solid vaporizable material, the heating temperature is chosen to not burn but only heat the vaporizable material. In case of a liquid vaporizable material, the heating temperature is chosen to vaporize the vaporizable material.

The aerosol generation device 12 extends along an axis X called hereinafter "device axis X". In the following description, the term "length" refers to a dimension of an element of the aerosol generation device 12 measured along the device axis X.

The aerosol generation device 12 is configured to operate between a vaping mode and a sensing mode. In the vaping mode, the aerosol generation device is configured to deliver aerosol to a user. In the sensing mode, the aerosol generation device is configured to sense at least a gas within an exhaled flow, exhaled by the user. These modes will be described in detail below.

The aerosol generation device 12 comprises a casing 18 and internal components arranged within the casing 18.

The internal components comprise a storage portion 20 configured to receive the consumable article 14, a gas-sensing compartment 22, preferably a sensing flow path 24 *via* which the compartment 22 is able to be in fluid communication with the storage portion 20, and a vaping flow inlet 26.

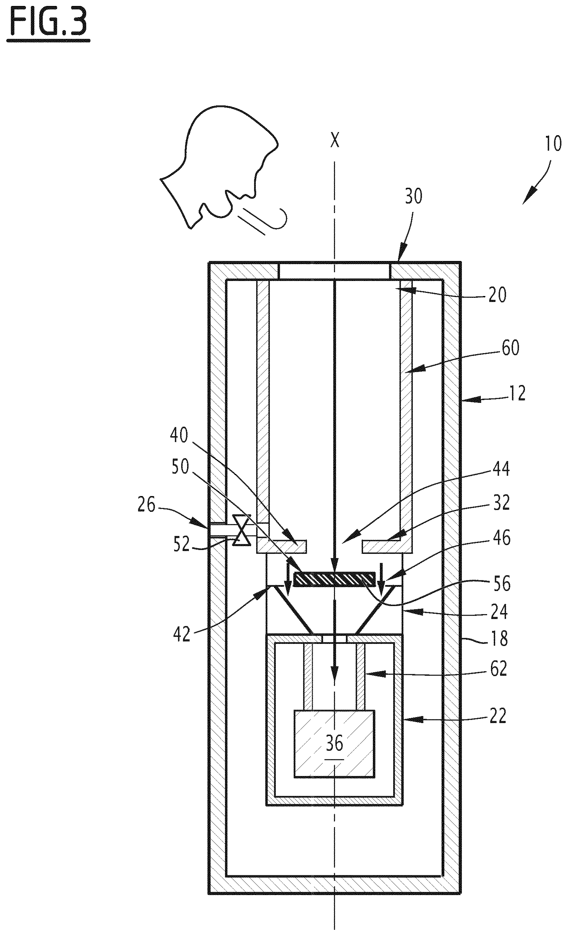

The storage portion 20 extends along the device axis X between an open end 30 and a bottom end 32. As shown on Figure 2, the storage portion 20 is configured to receive the consumable article 14 through the open end 30. In particular, in the vaping mode, the storage portion 20 is configured to conduct a vaping flow from the vaping flow inlet 26 to the open end 30 through the consumable article 14. The length of the storage portion 20 is for example smaller than the length of the consumable article 14 such that a mouth portion of the consumable article 14 protrudes from the open end 30 when the consumable article 14 is inserted into the storage portion 20. As shown on Figure 3, in the sensing mode, the storage portion 20 is configured to conduct the exhaled flow from the open end 30 to the gas-sensing compartment 22, for example when the storage portion 20 is empty of consumable article 14. In particular, in the sensing mode, the user exhales the exhaled flow within the storage portion 20 from the open end 30.

The gas-sensing compartment 22 is arranged at an opposite end of the device 12 along the device axis X with respect to the storage portion 20. It comprises a gas sensor 36 for sensing at least a gas. For instance, the gas sensed by the gas sensor 36 comprises a component chosen from the list comprising acetone, carbon dioxide and ethanol.

The gas sensor 36 is configured to generate sensing data representative of the exhaled flow. For example, said sensing data are representative of the sensed gas. They advantageously comprise information about its concentration within the exhaled flow.

The sensing flow path 24 extends from the storage portion 20 to the gas-sensing compartment and fluidically connects the storage portion 20 and the gas-sensing compartment 22. In the sensing mode, the exhaled flow exhaled by the user within the storage portion 20 flows from the storage portion 20 to the gas-sensing compartment 22 via the sensing flow path 24. The sensing flow path 24 is defined by a circumferential external wall 39 radially delimiting the sensing flow path 24, a first intersecting wall 40 and a second intersecting wall 42.

The first intersecting wall 40 separates the sensing flow path 24 from the storage portion 20 and, for instance, extends in a plane substantially perpendicular to the device axis X. The first intersecting wall 40 presents at least a first through-hole 44 connecting the storage portion 20 and the sensing flow path 24.

The second intersecting wall 42 is interposed between the first intersecting wall 40 and the gas-sensing compartment and, for instance, extends in a plane substantially perpendicular to the device axis X. The second intersecting wall 42 presents at least one second through-hole 46. According to the example of Figures 2 and 3, the second intersecting wall 42 presents several second through-holes 46 arranged at a radial periphery of the second intersecting wall 42.

The vaping flow inlet 26 connects the storage portion 20 and the outside of the device 12. As shown on Figures 2 and 3, the vaping flow inlet 26 is advantageously arranged at the bottom end 32 of the storage portion 20 and opens on a lateral wall of the device 12. In the vaping mode, when the user draws air at the open end 30, fresh air enters the storage portion 20 *via* the vaping flow inlet 26.

Optionally, the internal components of the device 12 further comprise a sensing valve 50 arranged between the gas-sensing compartment 22 and the storage portion 20 and a vaping valve 52 arranged between the vaping flow inlet 26 and the storage portion 20.

As shown on Figures 2 and 3, the sensing valve 50 controls the flow through the sensing flow path 24. The sensing valve 50 is movable between an open position (Figure 3) in which the gas-sensing compartment 22 is in fluid communication with the storage portion 20 and a closed position (Figure 2) in which the gas-sensing compartment 22 is isolated from the storage portion 20. Advantageously, the sensing valve 50 is in the open position in the sensing mode and in the closed position in the vaping mode. According to the examples of Figures 2 and 3, the sensing valve 50 comprises a plug 56 movable in translation along the device axis X between an upper position (Figure 2) and a lower position (Figure 3). When the sensing valve 50 is in the closed position, the plug 56 is in the upper position. In the upper position, the plug 56 seals the first through-hole 44 of the first intersecting wall 40 so that the sensing flow path 24 and the gas-sensing compartment 22 are isolated from the storage portion 20. When the sensing valve 50 is the open position, the plug 56 is in the lower position. In the lower position, the plug 56 is away from the first through-hole 44 so that the storage portion 20 communicates with the gas-sensing compartment 22 *via* the first through-hole 44 and the or each second through-hole 46.

With reference to Figures 2 and 3, the vaping valve 52 controls the flow through the vaping flow inlet 26. The vaping valve 52 is movable between an open position (Figure 2) in which the vaping flow inlet 26 is in fluid communication with the storage portion 20 and a closed position (Figure 3) in which the vaping flow inlet 26 is sealed. Advantageously, the vaping valve 52 is in the open position in the vaping mode and in the closed position in the sensing mode.

The internal components of the device 12 further comprises a first heating system 60 and, optionally, a second heating system 62.

The first heating system 60 is configured to heat at least partially the storage portion 20 until at least a first temperature to form aerosol on the open end 30 when the consumable article 14 is received in the storage portion 20. For example, the first temperature is substantially equal to the heating temperature. Advantageously, in the sensing mode, the first heating system 60 is further configured to heat the exhaled flow passing through the storage portion 20 until at least a second temperature. For instance, the second temperature is chosen to cause isolation of at least one predetermined component from the exhaled flow, that is at least one predetermined component from the sensed gas. Preferably, the second temperature is comprised between 80°C and 100°C, and advantageously substantially equal to 90°C. For instance, the first heating system 60 is arranged at least partially around the storage portion 20.

The second heating system 62 is configured to heat the exhaled flow entering the gas-sensing compartment 22 in the sensing mode, until at least the second temperature. For instance, the second heating system 62 is arranged at least partially within the gas-sensing compartment 22 and heats the exhaled flow before said flow reaches the gas sensor 36. In a variant, the second heating system 62 is arranged at least partially within the sensing flow path 24.

For example, the first heating system 60 and/or the second heating system 62 comprises a hollow cylindrical component, the exhaled flow flowing through the cylindrical component toward the gas-sensing compartment 22 in sensing mode. For instance, each of the first heating system 60 and the second heating system 62 comprises such a hollow cylindrical component. Advantageously, the cylindrical component comprises radially, inwardly to outwardly, a Teflon layer filled with a hydrocarbon filler, a copper layer and a heater film. For instance, the first heating system 60 and/or the second heating system 62 are electrically activated resistive heating elements.

With reference to Figure 1, optionally, the internal components comprise a control module, a switch system and a communication module (not shown).

The control module 66 is configured to control the device 12 between the sensing mode and the vaping mode. Particularly, in each of these modes, the control module 66 is configured to control the operation of the first heating system 60 and/or eventually, the second heating system 62.

The switch system 68 is designed to be manipulated by the user to command the control module 66. For example, the switch system 68 comprises a switch movable between a sensing position and a vaping position. When the switch is in the sensing position, the switch system 68 commands the control module 66 so that the device 12 is put into sensing mode. When the switch is in the vaping position, the switch system 68 commands the control module 66 so that the device 12 is put into vaping mode. For instance, the switch is a mechanical switch, an electronic switch or an electro-mechanical switch. Advantageously, the switch is a tactile switch.

Alternatively, the switch system 68 may be configured to detect whether the consumable article 14 is received in the storage portion 20 or not, and depending on it, activate automatically the vaping mode or the sensing mode.

As shown on Figure 1, the communication module 70 is connected to the gas sensor 36. The communication module 70 is configured to receive sensing data from the gas sensor 36. The communication module 70 is further configured to transmit said sensing data to the external device 1. As an example, the transmission of said sensing data to the external device 1 is carried out *via* Bluetooth.

The internal components may further comprise other elements performing different functionalities of the device. These other elements are known *per se* and are not be explained in further detail here.

As said above, the external device 1 is configured to communicate with the aerosol generation assembly 10, in particular with the aerosol generation device. For example, it comprises a display system. The external device 1 is advantageously configured to display information based on sensing data received from the communication module 70 on its display system. For example, said information concerns metabolic rates of the user, advices about exercises to perform, food or drink suggestions. The external device 1 is for example a connected device. It is for example a smartphone, a smartwatch or a tablet.

## Claims

1. An aerosol generation device (12) comprising:
- a storage portion (20) extending along a device axis (X) between an open end (30) and a bottom end (32);
- a first heating system (60) configured to heat at least partially the storage portion (20) until at least a first temperature to form aerosol on the open end (30); and
- a gas-sensing compartment (22), able to be in fluid communication with the storage portion (20) and comprising a gas sensor (36) for sensing at least a gas;
in a vaping mode, the storage portion (20) being configured to conduct a vaping flow from a vaping flow inlet (26) to the open end (30);
in a sensing mode, the storage portion (20) being configured to conduct an exhaled flow from the open end (30) to the gas-sensing compartment (22),
**characterized in that**:
- the storage portion (20) is configured to receive a consumable article (14) storing a vaporizable material;
- the first heating system (60) is configured to heat at least partially the storage portion (20) until the at least a first temperature to form aerosol on the open end (30) when the consumable article (14) is received in the storage portion (20);
- in the vaping mode, the storage portion (20) is configured to conduct the vaping flow from the vaping flow inlet (26) to the open end (30) through the consumable article (14);
- in the sensing mode, the storage portion (20) is configured to conduct the exhaled flow when the storage portion (20) is empty of consumable article (14).

2. The aerosol generation device (12) according to claim 1, further comprising a sensing valve (50) arranged between the gas-sensing compartment (22) and the storage portion (20);
the sensing valve (50) being movable between an open position in which the gas-sensing compartment (22) is in fluid communication with the storage portion (20) and a closed position in which the gas-sensing compartment (22) is isolated from the storage portion (20);
the sensing valve (50) being in the open position in the sensing mode and in the closed position in the vaping mode.

3. The aerosol generation device (12) according to claim 1 or 2, further comprising a vaping valve (52) arranged between the vaping flow inlet (26) and the storage portion (20);
the vaping valve (52) being movable between an open position in which the vaping flow inlet (26) is in fluid communication with the storage portion (20) and a closed position in which the vaping flow inlet (26) is sealed;
the vaping valve (52) being in the open position in the vaping mode and in the closed position in the sensing mode.

4. The aerosol generation device (12) according to any one of the preceding claims, further comprising a control module (66) and a switch system (68), the control module (66) being configured to control the device (12) between the sensing mode and the vaping mode, the switch system (68) being designed to be manipulated by a user to command the control module (66).

5. The aerosol generation device (12) according to any one of the preceding claims, wherein the storage portion (20) is configured to receive the consumable article (14) through the open end (30).

6. The aerosol generation device (12) according to any one of the preceding claims, wherein the vaping flow inlet (26) is arranged at the bottom end (32) of the storage portion (20).

7. The aerosol generation device (12) according to any one of the preceding claims, wherein the first heating system (60) is further configured to heat the exhaled flow passing through the storage portion (20) in the sensing mode, until at least a second temperature.

8. The aerosol generation device (12) according to any one of the preceding claims, further comprising a second heating system (62) configured to heat the exhaled flow entering the gas-sensing compartment (22) in the sensing mode, until at least a second temperature.

9. The aerosol generation device (12) according to claim 7 or 8, wherein the first heating system (60) and/or the second heating system (62) comprises a hollow cylindrical component, the exhaled flow flowing through the cylindrical component toward the gas sensor (36) in the sensing mode.

10. The aerosol generation device (12) according to claim 9, wherein the cylindrical component comprises, radially, inwardly to outwardly, a Teflon layer filled with a hydrocarbon filler, a copper layer and a heater film.

11. The aerosol generation device (12) according to any one of claims 7 to 10, wherein the second temperature is chosen to cause isolation of a at least one predetermined component from the exhaled flow, preferably the second temperature being comprised between 80°C and 100°C, and advantageously substantially equal to 90°C.

12. The aerosol generation device (12) according to any one of the preceding claims, further comprising a communication module (70) connected to the gas sensor (36), the gas sensor (36) being configured to generate sensing data representative of the exhaled flow, the communication module (70) being configured to transmit said sensing data to an external device (1).

13. The aerosol generation device (12) according to any one of the preceding claims, wherein the gas sensed by the gas sensor (36) comprises a component chosen from the list comprising acetone, carbon dioxide and ethanol.

14. The aerosol generation assembly, comprising:
- an aerosol generation device (12) according to any one of the preceding claims; and
- a consumable article (14) configured to operate with the aerosol generation device (12).

## Patentansprüche

1. Aerosolerzeugungsvorrichtung (12), umfassend:
- einen Speicherabschnitt (20), der sich entlang einer Vorrichtungsachse (X) zwischen einem offenen Ende (30) und einem unteren Ende (32) erstreckt;
- ein erstes Heizsystem (60), das konfiguriert ist, um den Speicherabschnitt (20) zumindest teilweise auf eine zumindest erste Temperatur zu erwärmen, um ein Aerosol an dem offenen Ende (30) zu bilden; und
- eine gaserfassende Kammer (22), die in der Lage ist, mit dem Speicherabschnitt (20) in Fluidverbindung zu sein und umfassend einen Gassensor (36) zum Erfassen mindestens eines Gases;
wobei der Speicherabschnitt (20) in einem Vaping-Modus konfiguriert ist, um einen Vaping-Strom von einem Vaping-Strömungseinlass (26) zu dem offenen Ende (30) zu leiten;
wobei der Speicherabschnitt (20) in einem Erfassungsmodus konfiguriert ist, um einen ausgeatmeten Strom von dem offenen Ende (30) zu der gaserfassenden Kammer (22) zu leiten,
**dadurch gekennzeichnet, dass**:
- der Speicherabschnitt (20) konfiguriert ist, um einen verbrauchbaren Artikel (14) aufzunehmen, der ein verdampfbares Material speichert;
- das erste Heizsystem (60) konfiguriert ist, um den Speicherabschnitt (20) zumindest teilweise auf die zumindest erste Temperatur zu erwärmen, um ein Aerosol an dem offenen Ende (30) zu bilden, wenn der Verbrauchsartikel (14) in dem Speicherabschnitt (20) aufgenommen ist;
- der Speicherabschnitt (20) in dem Vaping-Modus konfiguriert ist, um den Vaping-Strom durch den verbrauchbaren Artikel (14) von dem Vaping-Strömungseinlass (26) zu dem offenen Ende (30) zu leiten;
- der Speicherabschnitt (20) in dem Erfassungsmodus konfiguriert ist, um den ausgeatmeten Strom zu leiten, wenn der Speicherabschnitt (20) leer von dem Verbrauchsartikel (14) ist.

2. Aerosolerzeugungsvorrichtung (12) nach Anspruch 1, ferner umfassend ein Messventil (50), das zwischen der gaserfassenden Kammer (22) und dem Speicherabschnitt (20) angeordnet ist;
das Messventil (50) zwischen einer offenen Position, in der die gaserfassende Kammer (22) in Fluidverbindung mit dem Speicherabschnitt (20) ist, und einer geschlossenen Position, in der die gaserfassende Kammer (22) von dem Speicherabschnitt (20) isoliert ist, bewegbar ist;
wobei das Messventil (50) in der offenen Position in dem Erfassungsmodus und in der geschlossenen Position in dem Vaping-Modus ist.

3. Aerosolerzeugungsvorrichtung (12) nach Anspruch 1 oder 2, ferner umfassend ein Vaping-Ventil (52), das zwischen dem Vaping-Strömungseinlass (26) und dem Speicherabschnitt (20) angeordnet ist;
das Vaping-Ventil (52) zwischen einer offenen Position, in der der Vaping-Strömungseinlass (26) in Fluidverbindung mit dem Speicherabschnitt (20) ist, und einer geschlossenen Position, in der der Vaping-Strömungseinlass (26) abgedichtet ist, bewegbar ist;
wobei das Ventil (52) in der offenen Position in dem Vaping-Modus und in der geschlossenen Position in dem Erfassungsmodus ist.

4. Aerosolerzeugungsvorrichtung (12) nach einem der vorherigen Ansprüche, ferner umfassend ein Steuermodul (66) und ein Schaltsystem (68), wobei das Steuermodul (66) konfiguriert ist, um die Vorrichtung (12) zwischen dem Erfassungsmodus und dem Vaping-Modus zu steuern, und das Schaltsystem (68) konstruiert ist, um von einem Benutzer betätigt zu werden, um das Steuermodul (66) zu steuern.

5. Aerosolerzeugungsvorrichtung (12) nach einem der vorherigen Ansprüche, wobei der Speicherabschnitt (20) konfiguriert ist, um den Verbrauchsartikel (14) durch das offene Ende (30) aufzunehmen.

6. Aerosolerzeugungsvorrichtung (12) nach einem der vorherigen Ansprüche, wobei der Vaping-Strömungseinlass (26) an dem unteren Ende (32) des Speicherabschnitts (20) angeordnet ist.

7. Aerosolerzeugungsvorrichtung (12) nach einem der vorherigen Ansprüche, wobei das erste Heizsystem (60) ferner konfiguriert ist, um den Ausatmungsstrom, der den Speicherabschnitt (20) in dem Erfassungsmodus durchläuft, auf mindestens eine zweite Temperatur zu erwärmen.

8. Aerosolerzeugungsvorrichtung (12) nach einem der vorherigen Ansprüche, ferner umfassend ein zweites Heizsystem (62), das konfiguriert ist, um den in die gaserfassende Kammer (22) eintretenden Ausatmungsstrom in dem Erfassungsmodus auf mindestens eine zweite Temperatur zu erwärmen.

9. Aerosolerzeugungsvorrichtung (12) nach Anspruch 7 oder 8, wobei das erste Heizsystem (60) und/oder das zweite Heizsystem (62) eine hohle zylindrische Komponente umfasst, wobei der ausgeatmete Strom in dem Erfassungsmodus durch die zylindrische Komponente in Richtung des Gassensors (36) strömt.

10. Aerosolerzeugungsvorrichtung (12) nach Anspruch 9, wobei die zylindrische Komponente radial von innen nach außen eine mit einem Kohlenwasserstofffüllstoff gefüllte Teflonschicht, eine Kupferschicht und eine Heizfolie umfasst.

11. Aerosolerzeugungsvorrichtung (12) nach einem der Ansprüche 7 bis 10, wobei die zweite Temperatur gewählt ist, um eine Isolierung mindestens einer vorbestimmten Komponente aus dem ausgeatmeten Strom zu bewirken, wobei die zweite Temperatur vorzugsweise zwischen 80 °C und 100 °C liegt und vorteilhafterweise im Wesentlichen gleich 90 °C ist.

12. Aerosolerzeugungsvorrichtung (12) nach einem der vorherigen Ansprüche, ferner umfassed ein Kommunikationsmodul (70), das mit dem Gassensor (36) verbunden ist, wobei der Gassensor (36) konfiguriert ist, um Erfassungsdaten zu erzeugen, die repräsentativ für den ausgeatmeten Strom sind, und das Kommunikationsmodul (70) konfiguriert ist, um die Erfassungsdaten an eine externe Vorrichtung (1) zu übertragen.

13. Aerosolerzeugungsvorrichtung (12) nach einem der vorherigen Ansprüche, wobei das von dem Gassensor (36) erfasste Gas eine Komponente umfasst, die ausgewählt ist aus der Liste, umfassend Aceton, Kohlendioxid und Ethanol.

14. Aerosolerzeugungsanordnung, umfassend:
- eine Aerosolerzeugungsvorrichtung (12) nach einem der vorherigen Ansprüche; und
- einen Verbrauchsartikel (14), der für einen Betrieb mit der Aerosolerzeugungsvorrichtung (12) konfiguriert ist.

## Revendications

1. Dispositif de génération d'aérosol (12) comprenant :
- une partie de stockage (20) s'étendant le long d'un axe de dispositif (X) entre une extrémité ouverte (30) et une extrémité inférieure (32) ;
- un premier système de chauffage (60) configuré pour chauffer au moins partiellement la partie de stockage (20) jusqu'à au moins une première température pour former un aérosol sur l'extrémité ouverte (30) ; et
- un compartiment de détection de gaz (22), capable d'être en communication fluidique avec la partie de stockage (20) et comprenant un capteur de gaz (36) pour détecter au moins un gaz ;
dans un mode de vapotage, la partie de stockage (20) étant configurée pour acheminer un écoulement de vapotage depuis une entrée d'écoulement de vapotage (26) jusqu'à l'extrémité ouverte (30) ;
dans un mode de détection, la partie de stockage (20) étant configurée pour acheminer un écoulement exhalé de l'extrémité ouverte (30) jusqu'au compartiment de détection de gaz (22),
**caractérisé en ce que** :
- la partie de stockage (20) est configurée pour recevoir un article consommable (14) stockant un matériau vaporisable ;
- le premier système de chauffage (60) est configuré pour chauffer au moins partiellement la partie de stockage (20) jusqu'à l'au moins une première température pour former un aérosol sur l'extrémité ouverte (30) lorsque l'article consommable (14) est reçu dans la partie de stockage (20) ;
- dans le mode de vapotage, la partie de stockage (20) est configurée pour acheminer l'écoulement de vapotage depuis l'entrée d'écoulement de vapotage (26) jusqu'à l'extrémité ouverte (30) en passant par l'article consommable (14) ;
- dans le mode de détection, la partie de stockage (20) est configurée pour acheminer l'écoulement exhalé lorsque la partie de stockage (20) est vide de l'article consommable (14).

2. Dispositif de génération d'aérosol (12) selon la revendication 1, comprenant en outre une valve de détection (50) agencée entre le compartiment de détection de gaz (22) et la partie de stockage (20) ;
la valve de détection (50) étant mobile entre une position ouverte dans laquelle le compartiment de détection de gaz (22) est en communication fluidique avec la partie de stockage (20) et une position fermée dans laquelle le compartiment de détection de gaz (22) est isolé de la partie de stockage (20) ;
la valve de détection (50) se trouvant dans la position ouverte dans le mode de détection et dans la position fermée dans le mode de vapotage.

3. Dispositif de génération d'aérosol (12) selon la revendication 1 ou 2, comprenant en outre une valve de vapotage (52) agencée entre l'entrée d'écoulement de vapotage (26) et la partie de stockage (20) ;
la valve de vapotage (52) étant mobile entre une position ouverte dans laquelle l'entrée d'écoulement de vapotage (26) est en communication fluidique avec la partie de stockage (20) et une position fermée dans laquelle l'entrée d'écoulement de vapotage (26) est scellée ;
la valve de vapotage (52) se trouvant dans la position ouverte dans le mode de vapotage et dans la position fermée dans le mode de détection.

4. Dispositif de génération d'aérosol (12) selon l'une quelconque des revendications précédentes, comprenant en outre un module de commande (66) et un système de commutation (68), le module de commande (66) étant configuré pour commander le dispositif (12) entre le mode de détection et le mode de vapotage, le système de commutation (68) étant conçu pour être manipulé par un utilisateur afin de commander le module de commande (66).

5. Dispositif de génération d'aérosol (12) selon l'une quelconque des revendications précédentes, dans lequel la partie de stockage (20) est configurée pour recevoir l'article consommable (14) à travers l'extrémité ouverte (30).

6. Dispositif de génération d'aérosol (12) selon l'une quelconque des revendications précédentes, dans lequel l'entrée d'écoulement de vapotage (26) est agencée à l'extrémité inférieure (32) de la partie de stockage (20).

7. Dispositif de génération d'aérosol (12) selon l'une quelconque des revendications précédentes, dans lequel le premier système de chauffage (60) est en outre configuré pour chauffer l'écoulement exhalé traversant la partie de stockage (20) dans le mode de détection, jusqu'à au moins une seconde température.

8. Dispositif de génération d'aérosol (12) selon l'une quelconque des revendications précédentes, comprenant en outre un second système de chauffage (62) configuré pour chauffer l'écoulement exhalé entrant dans le compartiment de détection de gaz (22) dans le mode de détection, jusqu'à au moins une seconde température.

9. Dispositif de génération d'aérosol (12) selon la revendication 7 ou 8, dans lequel le premier système de chauffage (60) et/ou le second système de chauffage (62) comprend un composant cylindrique creux, l'écoulement exhalé s'écoulant à travers le composant cylindrique vers le capteur de gaz (36) dans le mode de détection.

10. Dispositif de génération d'aérosol (12) selon la revendication 9, dans lequel le composant cylindrique comprend, radialement, de l'intérieur vers l'extérieur, une couche de téflon remplie d'une charge d'hydrocarbure, une couche de cuivre et un film chauffant.

11. Dispositif de génération d'aérosol (12) selon l'une quelconque des revendications 7 à 10, dans lequel la seconde température est choisie pour provoquer l'isolement d'au moins un composant prédéterminé de l'écoulement exhalé, la seconde température étant de préférence comprise entre 80 °C et 100 °C, et avantageusement sensiblement égale à 90 °C.

12. Dispositif de génération d'aérosol (12) selon l'une quelconque des revendications précédentes, comprenant en outre un module de communication (70) relié au capteur de gaz (36), le capteur de gaz (36) étant configuré pour générer des données de détection représentatives de l'écoulement exhalé, le module de communication (70) étant configuré pour transmettre lesdites données de détection à un dispositif externe (1).

13. Dispositif de génération d'aérosol (12) selon l'une quelconque des revendications précédentes, dans lequel le gaz détecté par le capteur de gaz (36) comprend un composant choisi dans la liste comprenant l'acétone, le dioxyde de carbone et l'éthanol.

14. Ensemble de génération d'aérosol, comprenant :
- un dispositif de génération d'aérosol (12) selon l'une quelconque des revendications précédentes ; et
- un article consommable (14) configuré pour fonctionner avec le dispositif de génération d'aérosol (12).
